# EUROPEAN PATENT APPLICATION

(11) **EP 4 234 082 A1**
(43) Date of publication of application: **30.08.2023**
(21) Application number: 22158823.9
(22) Date of filing: 25.02.2022
(51) Int. Cl.: B01J 23/755, B01J 21/16, B01J 37/08, B01J 35/10, B01J 37/18, B01J 37/02, C07C 1/12

(54) **A METHOD FOR PREPARING A NICKEL-BASED METHANATION CATALYST ON A POROUS CLAY HETEROSTRUCTURE SUPPORT WITH A HIGH SPECIFIC SURFACE AREA, AND A NICKEL-BASED METHANATION CATALYST THUS OBTAINED**

(71) Applicant: Lietuvos Energetikos Institutas, 44403 Kaunas (LT)
(72) Inventor: TAMOSIUNAS, Andrius, 44403 Kaunas (LT); LUKOSIUTE, Stase Irena, 44403 Kaunas (LT); BALTUSNIKAS, Arunas, 44403 Kaunas (LT); CESNIENE, Jurate, 44403 Kaunas (LT); ZAKARAUSKAS, Kestutis, 44403 Kaunas (LT); STRIUGAS, Nerijus, 44403 Kaunas (LT)
(74) Representative: Pranevicius, Gediminas

(57) **Abstract**

The invention relates to a method for preparing a Ni-based methanation catalyst deposited on a porous clay heterostructure support with a high specific surface area (PCH-HSA) formed by a chemical method. During the first step of the synthesis, modification of sodium bentonite with quaternary ammonium salt with molecule of branched structure, high molecular weight, and long alkyl chains (C16-C18) is performed. The modifier is intercalated into the interlayer space of silicate (sodium bentonite) and expands the distance between the tetrahedral layers up 3.9 nm. At the second step, long alkyl chain primary neutral amine (C18) together with silicate source (tetraethylorthosilicate) is intercalated to the expanded interlayer of the modified silicate. Hydrolysis and polycondensation reaction of silicate source take place. PCH-HSA precursor is formed which is calcined at 640 °C for 5 hours. After the calcination, the PCH-HSA catalyst support with specific surface area S_{BET} (up to 1000-1100 m²/g), total pore volume (up to 1.15 cm³/g) and 4.09 nm interlayer spacing is formed. Then, a Ni catalyst is prepared by wetness impregnation of PCH-HSA with aqueous solutions of nickel nitrate with good dispersion of Ni nanoparticles (18 nm) on the PCH-HSA support. The Ni/PCH-HSA catalyst is characterized by high CO₂ conversion of 94*%*, synthetic bio-CH₄ yield of 88.6*%* and selectivity of 94*%*, respectively.

## Description

### FIELD OF INVENTION

The present invention relates to a field of catalysts and namely to a method for preparing a Nickel catalyst deposited on a high specific surface area porous clay heterostructure support (PCH-HSA) used for carbon dioxide/carbon monoxide (CO₂/CO) hydrogenation (Sabatier methanation reaction). The basic idea lies in optimization of production of Ni catalyst support using a two-step synthesis approach, i.e. modification of layered silicate sodium bentonite with quaternary onium ion (Arquad 2HT-75), and synthesis of porous clay heterostructure on the base of modified bentonite using neutral amine and silicate source such as tetraethylorthosilicate (TEOS).

### BACKGROUND OF THE INVENTION

The invention would address catalytic CO₂/CO and green (or blue) hydrogen utilization to synthetic bio-methane, waste-to-energy (WtE), power-to-X (PtX) and efficient biomass/waste management processes. From the energetic and commercial point of view, methane is an excellent cheaper fuel with relatively high volumetric and gravimetric chemical energy density, very well developed natural gas grid infrastructure and/or could be used as fuel in transportation sector. The properties of synthetic bio-methane produced via catalytic Sabatier reaction are equal to natural gas and thus it could be directly used as a substitute fuel or mixed with natural gas at any proportions from 0 to 100% and injected into the grid.

Usually, transition metals such as Ni, Ru, Rh, Pd and Pt are generally used as catalytic material for methanation in the form of metal nanoparticles (MNP) deposited on various supports (MNP/support). The support plays a key role on the activity and stability of the catalyst.

Presently, catalyst supports are used in methanation reaction:
1. Single component of metals oxides: SiO₂, TiO₂, ZrO₂, CeO₂, Al₂O₃, MgO, MnO, MnO₂, Mn₂O₃, and Mn₃O₄ with different crystal phases and types.
2. Modified oxides: CeO₂-Al₂O₃, CeO₂-ZrO₂-Al₂O₃, CaO-Al₂O₃, and MgO-SiO₂.
3. Metal-organic frameworks (MOFs): MOF-199, UiO-66, UiO-67.
4. Zeolites: USY, MFI and BEA types.
5. Mesoporous silica: MCM-41, SBA-15.
6. Carbon materials: carbon nanotubes CNT, ordered mesoporous carbon OMC, exfoliated graphitic carbon nitride.

However, these materials used as supports have some disadvantages:
- relatively small specific surface area and total pore volume;
- interaction between catalyst and support is weak and the catalyst is easily deactivated due to aggregation of its particles under high temperatures and carbon deposition;
- highly expensive;
- unstable pore structure at high temperature;
- necessity to modify the support due to enhanced CO₂ adsorption capacity;
- blockage of active sites during methanation reaction.

Therefore, new and more efficient supports are always sought.

Ni based catalysts have been mostly investigated due to high catalytic activity and selectivity towards methane production as well as from the economic point of view as a cheaper material. However, it is important to obtain a more efficient catalyst with a support for the CO₂/CO hydrogenation. In industry most commonly used commercial Ni/Al₂O₃ and Ni/CaO,Al₂O₃ (JM57-4Q) catalysts have CO₂ conversion 70-82%, CH₄ yield 62-65%, selectivity 82-95%, respectively (Miguel C.V. et al., J. CO2 Util., 2018, 25, 128-136).

Some metals or metal oxides deposited on porous clay heterostructure supports were used for oxidative dehydrogenation of ethane (Solsona B. et al., Catal. Sci. Technol., Iss., 2016, 6, 3419-3429; NiO), reduction of NO with C₃H₆ (Kashif M. et al., Vacuum, 2022, 198, 110879; Mn), synthetic esters production (Alves Saboya R. M. et al., Appl. Clay Sci., 2016, 124-125, 69-78; WO₃), preferential CO oxidation (Cecilia, J.A. et al., Catal. Today, 2015, 253, 126-136; CuO-CeO₂), etc. However, according to available scientific literature and patents screen in databases, nickel catalyst deposited on PCH-HSA has not been used for CO₂/CO hydrogenation yet.

The technology described herein allows optimization of the synthesis of a novel Ni catalyst deposited on a porous clay heterostructure support with a high specific surface area formed by chemical methods for maximum CO₂/CO conversion to synthetic bio-methane.

### SUMMARY OF THE INVENTION

The present invention relates to a method for preparing a Ni-based methanation catalyst wherein nickel metal nanoparticles are deposited on the novel support made of porous high specific surface area (S_{BET}) and pore volumes clay heterostructure formed by chemical method and used for Sabatier reaction producing bio-methane and water. New Ni/PCH-HSA catalyst is characterized by CO₂ conversion of 94%, high selectivity of 94%, CH₄ yield of 88.6% and does not require expensive catalytic materials such as Ru, Pt, and Pd. Moreover, none of the above mentioned disadvantages of the catalyst supports was found to be typical to the PCH-HSA support.

During the first step of the PCH-HSA support synthesis, i.e. the cationic exchange reaction, a modified bentonite is prepared. The organic compound - Quaternary ammonium salt with high molecular weight and branched structure of molecule - is intercalated into the interlayer of sodium bentonite and expands it from 1.25 nm up to 3.9 nm.

In the second step, neutral amine (octadecylamine) together with silicate source (tetraethylorthosilicate) is intercalated to the expanded interlayer space of modified silicate. Hydrolysis and polycondensation reaction of silicate source take place. Porous clay heterostructure precursor is formed, which is washed with methanol and calcined at 640 °C. After the calcination, the PCH-HSA with S_{BET} up to 1000-1100 m²/g and a total pore volume of 1.15 cm³/g is formed.

The last step is catalyst preparation by the 48 hours wetness impregnation of PCH-HSA in the saturated aqueous solution of nickel nitrate hexahydrate and drying at 100 °C for 24 hours. The heating at 450 °C of the impregnated PCH-HSA is used in order to complete the decomposition of nickel nitrate and to form NiO. Finally, the conversion of CO₂ and the yield of bio-methane over Ni/PCH-HSA are tested. Prior to the methanation reaction, the Ni/PCH-HSA catalyst is reduced according to the standard procedure. The high surface area of the support promotes good dispersion of the nickel metal nanoparticles (18 nm) over the surface of the PCH-HSA catalyst support.

The Ni/PCH-HSA catalyst is tested at a molar H₂ and CO₂ ratio of 4:1 at 400 °C when the loading of nickel is between 5-30 wt% and the content of the PCH-HSA carrier is between 70-95 wt%. With the increase of Ni loading from 5 to 30 wt%, the CO₂ conversion increased from 19 up to 94%, selectivity from 18.6 up to 94%, and bio-methane yield from 3.44 up to 88.6%, respectively.

Industrial nickel based catalyst deposited on Al₂O₃ support with different Ni loading has CO₂ conversion of 70-82%, CH₄ yield of 62-65%, and selectivity of 82-95%, respectively (Miguel C.V. et al., J. CO2 Util., 2018, 25, 128-136).

### BRIEF DESCRIPTION OF THE DRAWINGS

The present invention is described with reference to the accompanying drawings, wherein:
FIG. 1 depicts the powder X-ray diffraction patterns that demonstrate the formation of modified sodium bentonite after the cation exchange reaction. The distance between the tetrahedral layers of initial bentonite expands from 1.25 nm up to 3.9 nm after modification.
FIG. 2 depicts the powder X-ray diffraction patterns which show the formation of the crystalline structure of the PCH with the interlayer space of d = 4.09 nm.
FIG. 3 displays the N₂-gas adsorption/desorption isotherms of the initial sodium bentonite and PCH-HSA after the calcination, which is used for the specific surface area measurement and for the calculation of pore size distribution.
FIG. 4 depicts the pore size distribution of the initial bentonite and calcined PCH-HSA showing the formation of well defined micropore sizes of 1.3 nm and mesopores of 2.4-3.4 nm.
FIG. 5 depicts the powder X-ray diffraction patterns, which show of crystalline structure of the Ni-PCH-HSA catalyst with the crystallite size of 18 nm.

### DETAILED DESCRIPTION OF THE INVENTION

The invention relates to a method for preparing a Ni-based methanation catalyst wherein nickel metal nanoparticles are deposited on the novel support made of porous high specific surface area (S_{BET}) and pore volumes clay heterostructure formed by chemical method and used for Sabatier reaction producing synthetic bio-methane and water. The basic idea lies in optimization of production of Ni catalyst support using a two-step synthesis approach, i.e. modification of layered silicate sodium bentonite with quaternary onium ion (Arquad 2HT-75), and synthesis of porous clay heterostructure on the base of modified bentonite using neutral amine and silicate source such as tetraethylorthosilicate (TEOS). New Ni/PCH-HSA catalyst is characterized by CO₂ conversion of 94%, high selectivity of 94%, CH₄ yield of 88.6% and does not require expensive catalytic materials such as Ru, Pt, and Pd. None of the above mentioned disadvantages of the catalyst supports was found to be typical to the PCH-HSA support.

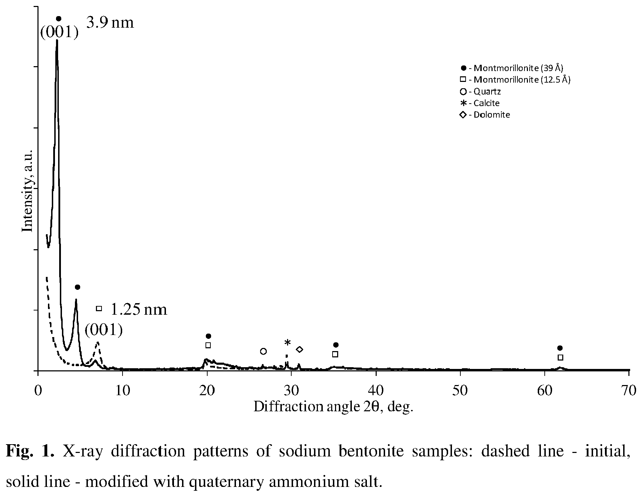

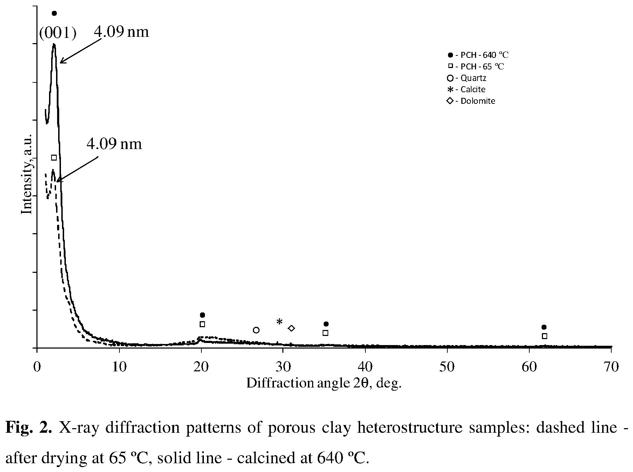

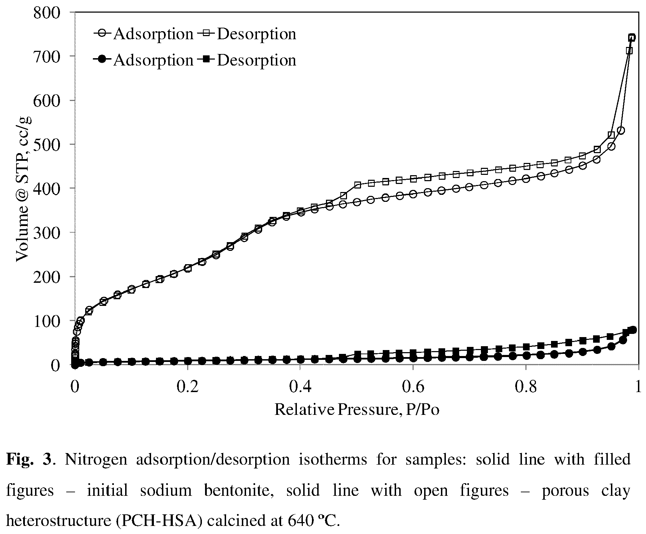

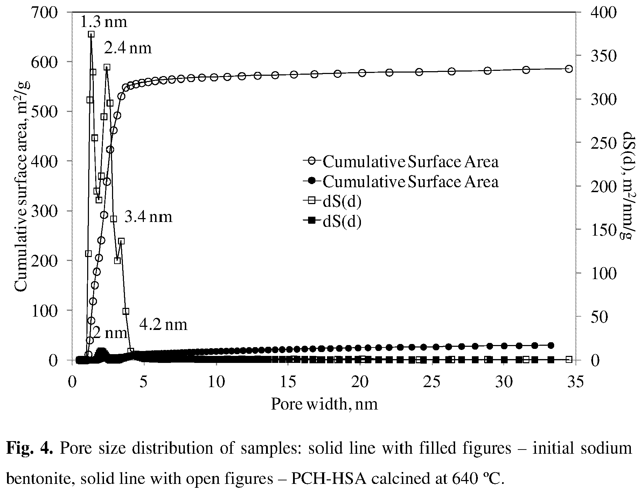

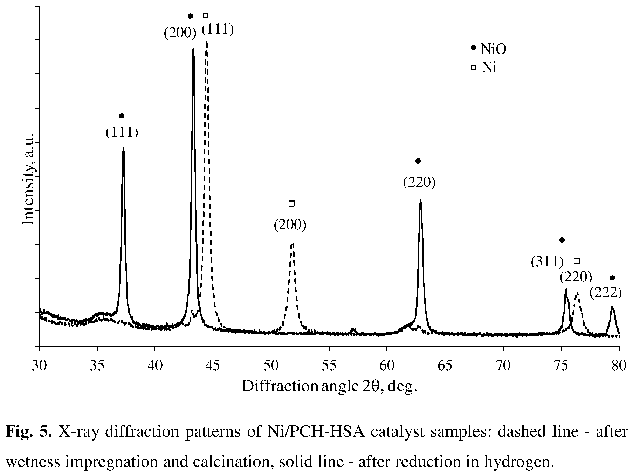

A method for preparing the Ni/PCH-HSA catalyst involves the following steps:
1. Modification of sodium bentonite. 4 wt% dispersion in water sodium bentonite (CEC 105meq/100g) and quaternary ammonium salt (quat's, dihydrogenated tallow dimethylammonium chloride, Arquad 2HT-75) with high molecular weight (M = 576 g/mol), branched molecule structure with long alkyl chains (C16-C18) are heated in closed vessels up to 60 °C. Then both components are mixed and heated for 0.5 hour at 60 °C at speed rate of 60 rpm. The concentration of quat's is 1.5-1.65 the cation exchange capacities (CEC) of bentonite. Modified bentonite (MB) is produced during the cation exchange reaction between sodium bentonite and quaternary ammonium salt. When the concentration of the modifier exceeds the CEC of the layered silicate, the modifier not only intercalates to the interlayer space of bentonite, but also covers and hydrophobizes the surface of plates. This facilitates next ongoing penetration of tetraethylorthosilicate (TEOS) - the source of inorganic oxide - into the interlayer. The reaction product is filtered and washed with water. The XRD analysis of initial and solid intermediate-product (modified bentonite) is presented in Fig. 1. The distance between the tetrahedral layers of bentonite (montmorillonite) expands from 1.25 nm up to 3.9 nm.
2. 15 wt% dispersion of the MB in water is prepared by mixing for 2 hours with the speed rate of 120 rpm under the ambient conditions. Neutral amine (NA) - octadecylamine (C18) - is dissolved in methanol (saturated solution) and slowly poured into the dispersion of bentonite by mixing with the speed rate of 120 rpm for 0.5 hour. Then TEOS is added and the composition is stirred for 3 hours with the speed rate of 180 rpm under the ambient conditions. In order to obtain the high S_{BET} up to 1000-1100 m²/g, the molar ratio of NA and TEOS should be 8.0: 8.13, respectively. Micropores and mesopores are formed because of the use of longer alkyl chain (C18) neutral amine. The basal Bragg spacing of PCH-HSA (air dried) obtained by the XRD is 4.09 nm and is shown in Fig. 2. The remaining only one basal reflection of (001, d = 4.09 nm) indicate the partially disruption (delamination) of layered structure of modified clay and the formation of silica network between the clay layers.
3. N₂-gas adsorption/desorption isotherms of the PCH-HSA are recorded in liquid nitrogen at temperature of -196 °C. The specific surface area is determined and calculated by multi-point BET Plot method. According to the isotherms presented in Fig. 3, the S_{BET} of 1000-1100 m²/g is calculated. A high steep uptake at very low P/P₀ in the PCH-HSA isotherm indicates about the large amount of micropores formation. Micropores and mesopores are formed because of the use of longer alkyl chain (C18) neutral amine. The total pore volume up to 1.15 cm³/g, the micropores size of 1.3 nm and the mesopores size in the range of 2.4 and 3.4 nm are determined. The total pore volume increased from 0.12 cm³/g of the initial bentonite to 1.15 cm³/g of the PCH-HSA, respectively. The considerable increase of the S_{BET} value from 34 m²/g of raw bentonite to 1000 m²/g of PCH-HSA is obtained. The pore size distribution is determined by N₂ at -196 °C on carbon slit pore, QSDFT equilibrium model (Quantachrome. ASiQwin) and is presented in Fig. 4.
4. Porous clay heterostructure support (the calcined PCH-HSA) is used for catalyst preparation by the wetness impregnation of PCH-HSA with aqueous solution of nickel nitrate hexahydrate for 48 hours, drying for 24 hours at 100 °C and calcination at 450 °C with the heating rate of 1 °C/min for 5 hours in order to complete the decomposition of the nickel nitrate and to form NiO. High surface area of the catalyst support is an important factor in the methanation process leading to a higher dispersion of nickel metal nanoparticles (18 nm) on the surface. The X-ray diffraction analysis of the Ni/PCH-HSA is depicted in Fig.5. The crystallite size estimation of deposited Ni catalyst was performed using TOPAS-4.1 - the X-ray diffraction profile analysis program.
5. The hydrogenation of carbon dioxide is carried out using a conventional fixed bed tube reactor. The mixture of H₂ and CO₂ prepared by taking pure gases from cylinders. The required H₂:CO₂ (4:1) ratios are adjusted with mass flow controllers. The prepared mixture is fed into the gas heater to be heated to a temperature of approximately 300 °C. The heated gas is then directed to the methanation process reactor. For the pre-heating and simultaneous cooling of the reaction zone, a hot thermal-oil system is used. In the hydrogenation reactor, the Ni/PCH-HSA catalyst is placed between two layers of quartz wool. Prior to the reaction, the catalyst is reduced under hydrogen atmosphere at 627 °C for 2 h. After the reaction, the product gas enters a water-cooled condenser. Additionally, in order to increase the process efficiency, a gas pressure regulator is installed behind the condenser to maintain a constant reaction pressure of 10 bars. Finally, the reaction products are fed to a semi-online gas analysis system, the Agilent Micro GC 490 gas chromatograph. With the increase of Ni loading from 5 to 30 wt%, the CO₂ conversion increased from 19 up to 94%, selectivity from 18.6 up to 94%, and methane yield from 3.44 up to 88.6%, respectively.

Product has the following advantages:
1. Quaternary ammonium salt (dihydrogenated tallow dimethylammonium chloride) - modifier of sodium bentonite - has molecule of branched structure, long alkyl chains (C16-C18) and high molecular weight (M = 576 g/mol). Because the modified bentonite obtained high interlayer space (3.9 nm), the intercalation of other reagents to the tetrahedral layers is more accessible.
2. The PCH-HSA support of the novel catalyst has high specific surface area S_{BET} (up to 1000-1100 m²/g), total pore volume (up to 1.15 cm³/g), good dispersion of Ni nanoparticles (18 nm). None of the above mentioned disadvantages of the catalyst supports was found to be typical to the PCH-HAS support.
3. The Ni/PCH-HSA catalyst is characterized by high CO₂ conversion of 94%, synthetic bio-CH₄ yield of 88.6% and selectivity of 94%, respectively.

## Claims

1. A method for preparing a Ni-based methanation catalyst on a porous clay heterostructure support with a high specific surface area (PCH-HSA), **characterized by** the steps of:
a) modifying the layered silicate sodium bentonite with quaternary ammonium salt with high molecular weight and branched, long alkyl chains (C16-C18) molecule structure;
b) intercalating the neutral amine together with silicate source into the expanded interlayer space of bentonite, where hydrolysis and polycondensation reactions take place, thus forming the PCH-HSA precursor;
c) calcining the PCH-HSA precursor at 640 °C for 5 hours at a heating rate 1 °C/min, thus preparing the PCH-HSA;
d) using the PCH-HSA support for catalyst preparation by wetness impregnation of PCH-HSA with aqueous solution of nickel nitrate hexahydrate for 48 hours, drying at 100 °C for 24 hours and heating at 450 °C for 5 hours in order to complete the decomposition of the nickel nitrate and formation of NiO.

2. A Ni-based methanation catalyst on a porous clay heterostructure support with a high specific surface area (PCH-HSA), produced according to steps a) to d), having an increased Ni loading from 5 to 30 wt% on the PCH-HSA catalyst support, CO₂ conversion of 94%, synthetic bio-CH₄ yield of 88.6% and selectivity of 94%, respectively.
